# EUROPEAN PATENT SPECIFICATION

(11) **EP 3 398 959 B1**
(45) Date of publication and mention of the grant of the patent: **25.11.2020**
(21) Application number: 16880982.0
(22) Date of filing: 16.12.2016
(51) Int. Cl.: C07K 14/605, C07K 1/06

(54) **METHOD FOR PREPARING LIXISENATIDE**
VERFAHREN ZUR HERSTELLUNG VON LIXISENATID
MÉTHODE DE PRÉPARATION DU LIXISÉNATIDE

(30) Priority: 31.12.2015 CN 201511032042
(43) Date of publication of application: 07.11.2018
(73) Proprietor: Hybio Pharmaceutical Co., Ltd., Guangdong 518057 (CN)
(72) Inventor: CHEN, Xinliang, Shenzhen City Guangdong 518057 (CN); MI, Pengcheng, Shenzhen City Guangdong 518057 (CN); TAO, Anjin, Shenzhen City Guangdong 518057 (CN); YUAN, Jiancheng, Shenzhen City Guangdong 518057 (CN)
(74) Representative: HGF
(86) International application number: PCT/CN2016/110374
(87) International publication number: WO 2017/114192

(56) References cited:
- WO-A1-2013/098191
- WO-A1-2014/147124
- CN-A- 102 558 338
- CN-A- 103 709 243
- CN-A- 103 819 553
- CN-A- 104 211 801
- CN-A- 104 844 706
- CN-A- 104 844 706

## Description

### FIELD

The invention relates to the technical field of polypeptide drug synthesis, in particular to a method for preparing lixisenatide.

### BACKGROUND

Lixisenatide is a glucagon-like peptide-1 (GLP-1) receptor agonist. A novel GLP-1 analogue, which is synthesized through modification on the basis of the six lysine residues in the C-terminal of GLP-1 analogue Exendin-4 with an activity of lowering glucose, is resistant to the degradation of dipeptidyl peptidase IV (DPP4) *in vivo.* Lixisenatide has the structure as shown in Formula I: 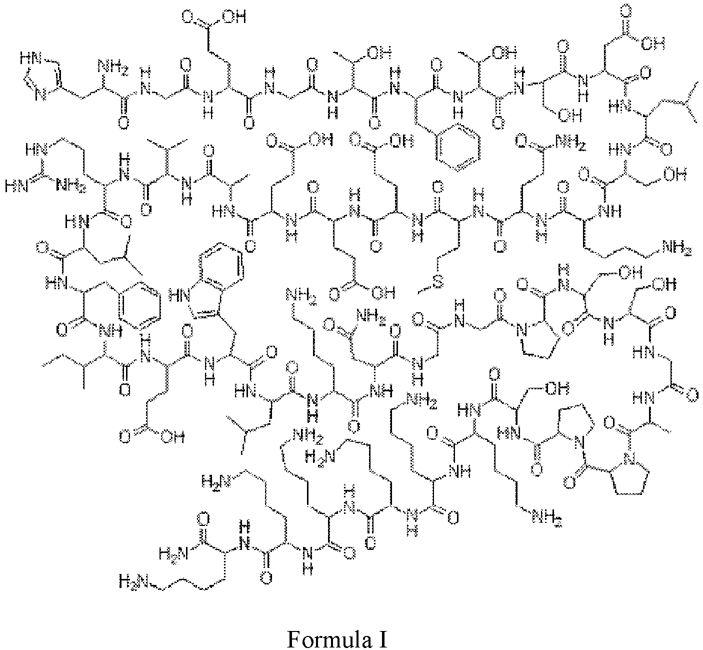 with the peptide sequence:

H-His-Gly-Glu-Gly-Thr-Phe-Thr-Ser-Asp-Leu-Ser-Lys-Gln-Met-Glu-Gl
u-Glu-Ala-Val-Arg-Leu-Phe-Ile-Glu-Trp-Leu-Lys-Asn-Gly-Gly-Pro-Ser-Ser-
Gly-Ala-Pro-Pro-Ser-Lys-Lys-Lys-Lys-Lys-Lys-NH2

As one of the GLP-1 analogues, lixisenatide is suitable as an adjuvant therapy to improve blood sugar control for being used in patients with type 2 diabetes who are administrated with metformin, sulfonylureas, thiazolidinediones, a combination of metformin and sulphonylureas, a combination of metformin and thiazolidinediones but not able to effectively control their blood glucose.

Currently, a conventional method for synthesizing lixisenatide is the solid-phase peptide synthesis (SPPS) method, which has the characteristics of simple operation and low equipment requirements. However, because lixisenamine is composed of 44 amino acids, the yield of the solid-phase peptide synthesis method is low, and the peptides on the synthetic resin tend to form secondary structure and bend, resulting in the block of the reaction site. The resulting lixisenatide crude peptide has a low purity and complicated impurities, making it difficult to be purified and obtain lixisenatide with a higher purity. CN104844706 is directed to a method for synthesizing lixisenatide. The method comprises the following steps: synthesizing amino acid fragments of first and second sites, amino acid fragments of third and fourth sites, amino acid fragments of 29th and 30th sites, amino acid fragments of 32nd and 33rd sites and amino acid fragments of 34th and 35th sites of lixisenatide at first, and then performing synthesis by adopting two manners including fragment synthesis and one-by-one synthesis according to a peptide sequence from an end C to an end N of a main chain of lixisenatide. According to the method, lixisenatide is prepared by adopting specific segments and one-by-one synthesis, and on the premise of ensuring relatively high overall yield and purity, the impurities including [Di-His1]-lixisenatide, [Di-Gly]-lixisenatide, [Di-Ser33]-lixisenatide, [Di-Ala35]-lixisenatide and the like are reduced by adopting a special synthesis strategy, so that the quality of a lixisenatide product is improved. WO 2013/098191 is directed to a dipeptide comprising a non-proteogenic amino acid, methods of making such and methods of using said dipeptide in a process of making a polypeptide or protein comprising one or more non-proteogenic amino acids. WO 2014/147124 is directed to a method of synthesizing a peptide product comprising at least one hydantoin group. The peptide product may be used as a reference material for the quality control of pharmaceutical peptides, particularly for the quality control of exendin peptides. Further, hydantoin building blocks, a method for manufacturing such building blocks and their use for the synthesis of peptide products are described.

For solving the problem of amino acids with difficulty in coupling, the segment synthesis is a common way used in the solid-phase synthesis. In the segment synthesis, the sequence with difficulty in coupling is first prepared as a fully protected peptide segment, and then the peptide segment as a whole is directly coupled to a designated peptide resin according to the amino acid sequence, thereby avoiding the amino acid site with difficulty in coupling, and avoiding the side effect such as defects produced in the coupling of amino acids one by one, therefore reducing the difficulty of purification and obtaining crude peptide with relatively high purity. However, the segment synthesis uses, besides the conventional resin, a large amount of expensive 2-CTC resin to prepare the segment peptide resin. In addition, the 2-CTC resin has high acid sensitivity, and the peptide is easily detached from the resin during coupling, causing a relatively low yield of the prepared segments. Also, the operation steps are relatively complicated, and the production cost is greatly increased.

### SUMMARY

In view of this, the technical problem to be solved by the present invention is to provide a method for preparing Lixisenatide, which has simple operation, high total yield, and high product purity.

The present invention provides a method for preparing Lixisenatide, comprising:
step 1: synthesizing Fmoc-Lys-resin by solid-phase synthesis;
step 2: coupling an amino acid and a dipeptide to the Fmoc-Lys-resin according to the peptide sequence of lixisenatide to obtain lixisenatide peptide resin; wherein the dipeptide is selected from the group consisting of -Gly-Thr, -Phe-Thr, -Thr-Ser, -Leu-Ser, -Ser-Ser and -Pro-Ser;
step 3: cleaving the lixisenatide peptide resin to obtain lixisenatide.

In the method provided by the present invention, dipeptides corresponding to positions 4 and 5 (-Gly-Thr, denoted as dipeptide A), positions 6 and 7 (-Phe-Thr, denoted as dipeptide B), positions 7 and 8 (-Thr-Ser, denoted as dipeptide C), positions 10 and 11 (-Leu-Ser, denoted as dipeptide D), positions 32 and 33 (-Ser-Ser, denoted as dipeptide E) or positions 31 and 32, positions 37 and 37(-Pro-Ser, denoted as dipeptide F) of the lixisenatide peptide are used as raw materials for coupling, wherein the selected dipeptide is one or more of dipeptides A to F. Coupling at the sites where no dipeptide is used is performed by using single amino acids as raw materials. By using this method, the rotation of the peptide bond can be effectively prevented, the contraction and curling of the peptide chain is suppressed, so that active functional groups (primary amine) are fully exposed, thereby facilitating the coupling of the amino acid, and reducing the occurrence of defects and other side effects.

According to the present invention, coupling -Gly-Thr is performed by using Fmoc-Gly-Thr(PSI ME,ME Pro)-OH;
coupling -Phe-Thr is performed by using Fmoc-Phe-Thr(PSI ME,ME Pro)-OH;
coupling -Thr-Ser is performed by using Fmoc-Thr(tBu)-Ser(PSI ME,ME Pro)-OH;
coupling -Leu-Ser is performed by using Fmoc-Leu-Ser(PSI ME,ME Pro)-OH;
coupling -Ser-Ser is performed by using Fmoc-Ser(tBu)-Ser(PSI ME,ME Pro)-OH;
coupling -Pro-Ser is performed by using Fmoc-Pro-Ser(PSI ME,ME Pro)-OH;
wherein, Fmoc-Gly-Thr(PSI ME,ME Pro)-OH has the structure as shown in formula II-a;
Fmoc-Phe-Thr(PSI ME,ME Pro)-OH has the structure as shown in formula II-b;
Fmoc-Thr(tBu)-Ser(PSI ME,ME Pro)-OH has the structure as shown in formula II-c;
Fmoc-Leu-Ser(PSI ME,ME Pro)-OH has the structure as shown in formula II-d;
Fmoc-Ser(tBu)-Ser(PSI ME,ME Pro)-OH has the structure as shown in formula II-e;
Fmoc-Pro-Ser(PSI ME,ME Pro)-OH has the structure as shown in formula II-f,

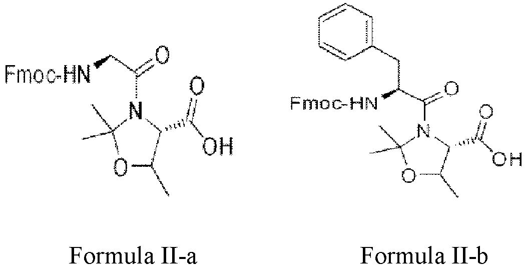

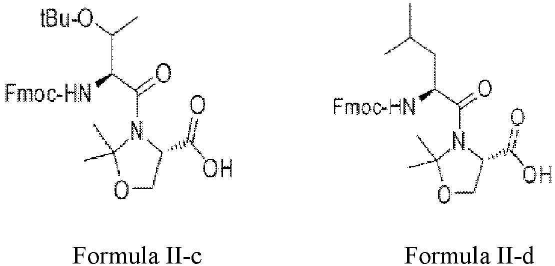

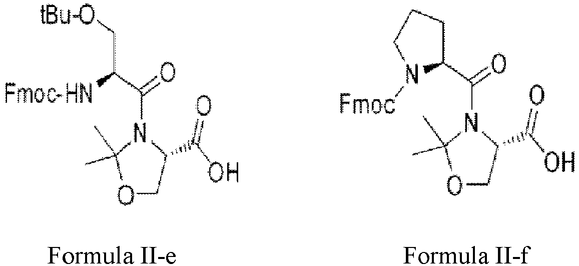

In the present invention, the resin of the Fmoc-Lys-resin is Rink Amide resin, Rink Amide-MBHA resin, Rink Amide-AM resin, or Siber resin; the protecting group of Lys is Boc.

Preferably, the degree of substitution of the Fmoc-Lys(Boc)-resin is 0.1 mmol/g to 0.6 mmol/g; more preferably, 0.2 mmol/g to 0.4 mmol/g.

In an embodiment of the present invention, the coupling agent for the coupling is a mixture of HOBt and DIC; wherein the molar ratio of HOBt to DIC is 1:1.

In the present invention, the steps of coupling comprise: removing Fmoc by deprotection, mixing the resin with the coupling agent in a mixture of DCM and DMF as the solvent, conducting coupling reaction.

Preferably, the molar ratio of the deprotected resin to the amino acid to be coupled is 1:3.

The coupling is repeated until the entire peptide chain is synthesized.

In the present invention, the condition of the coupling reaction is at room temperature for 2 hours. The room temperature is 10°C to 30°C.

Preferably, the coupling reaction is continued for additional 1 hour if the coupling reaction is incomplete.

In the present invention, after the completion of the coupling reaction, the resin is shrunk by methanol and dried under vacuum overnight.

In an embodiment of the present invention, the cleaving agent for the cleaving comprises TFA and component B; wherein the component B is selected from the group consisting of PhSMe, PhOMe, EDT, H2O, TIS and PhOH.

Preferably, the volume ratio of TFA, PhSMe, PhOMe, EDT, H₂O, TIS, PhOH in the cleaving agent is (80-90) : (0-5) : (0-3) : (0-5) : (0-5) : (0-2) : (0-5).

Preferably, the volume ratio of TFA, PhSMe, EDT, TIS, H₂O in the lysate is 84:5:5:5: 1.

Preferably, the volume ratio of TFA, PhSMe, EDT, H₂O in the cleaving agent is 91:3:3:3.

Preferably, the volume ratio of TFA, PhSMe, EDT, PhOMe in the cleaving agent is 90:5:3:2.

Preferably, the volume ratio of TFA, EDT, H₂O in the cleaving agent is 90:5:5.

Preferably, the volume ratio of TFA, PhSMe, PhOH, EDT, H₂O in the cleaving agent is 85:5:3:5:2.

In the present invention, the steps of the cleaving comprise: mixing the lixisenatide peptide resin with the cleaving solution, cleaving at room temperature for 2.5 h to 3 h, washing the resin with TFA, and precipitating with anhydrous diethyl ether to obtain a crude linear lixisenatide peptide.

Preferably, the precipitating with anhydrous diethyl ether is at a temperature of 0 ∼ 4°C.

Preferably, the mass-to-volume ratio of lixisenatide resin to cleaving solution is 1:8 ∼ 15.

In an embodiment of the present invention, step 3 is followed by steps of purification and salt conversion.

Specifically, the chromatography for purification is performed by using NOVASEP RP-HPLC system at a detection wavelength of 220 nm. The column is a reversed-phase C18 column, the mobile phase A is a TFA aqueous solution with a volume fraction of 0.1%, and the mobile phase B is acetonitrile. The elution gradient is 18% acetonitrile to 48% acetonitrile for 45 min, 48% isocratic elution for 15 min. After purification, a salt conversion chromatography is used for salt conversion.

Specifically, the salt conversion is to conver the lixisenatide into acetate, hydrochloride, citrate, phosphate, trifluoroacetate, sodium, potassium or ammonium salts.

In some embodiments, lixisenatide is prepared by introducing only dipeptide A, dipeptide C, dipeptide D, dipeptide E, dipeptide F.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Thr(tBu)-Ser(PSI ME, ME Pro)-OH, Fmoc-Phe-OH, Fmoc-Gly-Thr(PSI ME, ME Pro)-OH, Fmoc-Glu(OtBu)-OH, Fmoc-Gly-OH and Boc-His(Trt)-OH.

In some embodiments, lixisenatide is prepared by introducing only dipeptide A, dipeptide B, dipeptide D, dipeptide F.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME,ME Pro)-OH, Fmoc-Pro-OH, Fmoc-Ala-OH, Fmoc-Gly-OH, Fmoc-Ser(tBu)-OH, Fmoc-Pro-Ser(PSI ME,ME Pro)-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME,ME Pro)-OH, Fmoc-Gly-Thr(PSI ME,ME Pro)-OH, Fmoc-Glu(OtBu)-OH, Fmoc-Gly-OH and Boc-His(Trt)-OH.

In some embodiments, lixisenatide is prepared by introducing only dipeptide A, dipeptide B, dipeptide D, dipeptide E.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME,ME Pro)-OH, Fmoc-Gly-Thr(PSI ME,ME Pro)-OH, Fmoc-Glu(OtBu)-OH, Fmoc-Gly-OH and Boc-His(Trt)-OH.

In some embodiments, lixisenatide is prepared by introducing only dipeptide B, dipeptide D, dipeptide E.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME,ME Pro)-OH, Fmoc-Thr(tBu)-OH, Fmoc-Gly-OH, Fmoc-Glu(OtBu)-OH, Fmoc-Gly-OH and Boc-His(Trt)-OH.

In some embodiments, lixisenatide is prepared by introducing only dipeptide C, dipeptide E.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Thr(tBu)-Ser(PSI ME,ME Pro)-OH, Fmoc-Phe-OH, Fmoc-Thr(tBu)-OH, Fmoc-Gly-OH, Fmoc-Glu(OtBu)-OH, Fmoc-Gly-OH and Boc-His(Trt)-OH.

In some embodiments, lixisenatide is prepared by introducing only dipeptide E.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH and Boc-His(Trt)-OH.

In some embodiments, lixisenatide is prepared by introducing only dipeptide D.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH or Boc-His(Trt)-OH.

In some embodiments, lixisenatide is prepared by introducing only dipeptide A, dipeptide B, dipeptide D, dipeptide E, dipeptide F.

Specifically, the coupling is performed by coupling sequentially the followings: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME,ME Pro)-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME,ME Pro)-OH, Fmoc-Gly-Thr(PSI ME,ME Pro)-OH, Fmoc-Glu(OtBu)-OH, Fmoc-Gly-OH and Boc-His(Trt)-OH.

According to the peptide sequence of lixisenatide, specially protected serine dipeptide is used as a raw material and coupled to the peptide sequence which forms a ring-shaped structure similar to that of proline, therefore the rotation of a peptide bond can be effectively prevented, the contraction and curling of the peptide chain is suppressed, so that the active functional groups (primary amine) are fully exposed, thereby facilitating the coupling of the amino acid, and reducing the occurrence of defects and other side effects. According to the experiment, the yield of the refined peptide of the present invention is as high as 25.8% to 28.8%, which is superior to the stepwise coupling method (about 15.4%). At the same time, the purity of the fine peptide of the present invention is 99.3%. The purity is guaranteed while the yield is ensured by the method of the present invention.

### BRIEF DESCRIPTION OF THE DRAWINGS

Figure 1 shows the mass spectrum of the crude peptide prepared in Example 1.
Figure 2 shows the mass spectrum of the crude peptide prepared in Comparative Example 1.

### DETAILED DESCRIPTION OF EMBODIMENTS

The present invention provides methods for preparing lixisenatide. The method and application of the present invention have been defined in the claims and exemplified through the preferred embodiments.

The reagents or instruments used in the present invention are all common commercial products and are all available in the market.

Among them, the names and abbreviations of the materials are shown in Table 1: Table 1 Names and abbreviations of the materials

| | |
|---|---|
| Fmoc | 9-fluorenylmethyloxycarbonyl |
| Resin | Resin |
| tBu | Tert-butyl |
| OtBu | Tert-butoxy |
| Trt | Triphenylmethyl |
| DCM | Dichloromethane |
| DBLK | 20% hexahydropyridine/DMF solution |
| DIPEA | N,N-diisopropylethylamine |
| HOBt | 1 -hydroxybenzotriazole |
| HOAt | 1-hydroxy-7-azobenzotriazole |
| PyBOP | Benzotriazol-1-yl-oxytripyrrolidinyl hexafluorophosphate |
| DMSO | Dimethyl Sulfoxide |
| HATU | O-(7-azobenzotriazol-1-oxo)-N,N,N',N'-tetramethyluronium hexafluorophosphate |
| HPLC | High performance liquid chromatography |
| DMF | N,N-Dimethylformamide |
| TFA | Trifluoroacetic acid |
| HBTU | O-benzotriazole-N,N,N',N'-tetramethyluronium hexafluorophosphate |
| PyAOP | Triazolo[4,5-b]pyridin-3-oxy)tri-1-pyrrolidinyl hexafluorophosphate |
| PhOH | Phenol |
| PhOMe | Anisole |
| PheSMe | Thioanisole |
| EDT | 1,2-ethanedithiol |
| TIS | Triisopropylsilane |

In the following, the present invention will be further illustrated in combination with examples:

### Example 1

In this example, only dipeptide A, dipeptide C, dipeptide D, dipeptide E, and dipeptide F were introduced to prepare lixisenatide.

### 1.1 Preparation of Fmoc-Lys(Boc)-Rink Amide-MBHA Resin

380 g of dry Rink Amide-MBHA Resin (substitution degree of 0.45 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 238.9 g of Fmoc-Lys(Boc)-OH (510 mmol) and 72.4 g of HOAt (536 mmol) were dissolved in a mixed solvent of DMF and DCM. After the amino acid was dissolved, 67.5 g (535 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 421 g of Fmoc-Lys(Boc)-Rink Amide-MBHA Resin with a substitution degree detected of 0.406 mmol/g.

### 1.2 Preparation of lixisenatide peptide resin

421 g (230 mmol) of Fmoc-Lys(Boc)-Rink Amide-MBHA Resin with a substitution degree of 0.406 mmol/g prepared in method 1.1 was weighted and added into a solid-phase reaction column. The resin was washed twice with DMF, followed by swelling in DMF for 30 minutes. After the deprotection of Fmoc by DBLK, the resin was washed 6 times with DMF. 239.1 g (510 mmol) Fmoc-Lys(Boc)-OH, 72.2 g (536 mmol) HOBt, and 67.7 g (535 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Thr(tBu)-Ser(PSI ME, ME Pro)-OH, Fmoc-Phe-OH, Fmoc-Gly-Thr(PSI ME, ME Pro)-OH, Fmoc-Glu(OtBu)-OH, Fmoc-Gly-OH, and Boc-His(Trt)-OH. After the reaction was completed, the resin was shrunk by methanol and dried under vacuum overnight to give 2018 g of lixisenatide peptide resin.

### 1.3 Cleaving of lixisenatide peptide resin and preparation of refined peptide

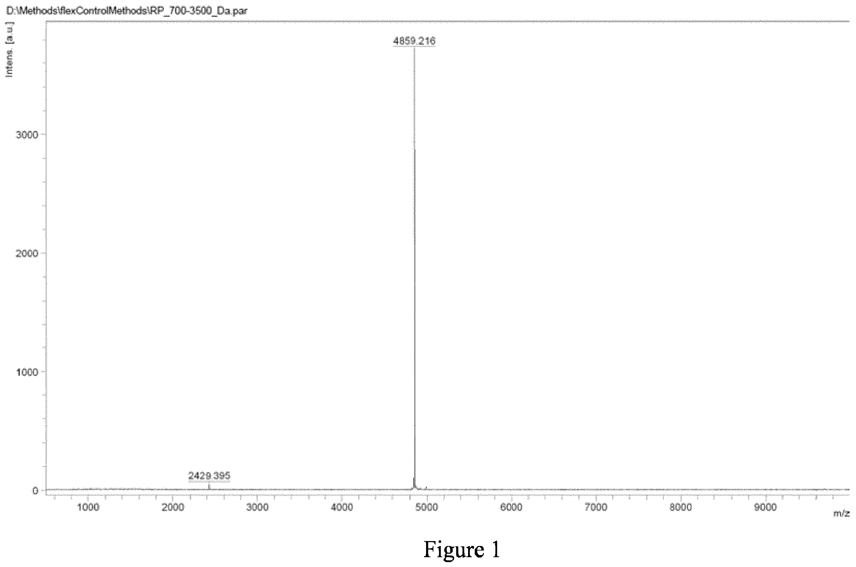

700 g of lixisenatide peptide resin obtained in 1.2 was placed in a cleaving reactor and a cleaving agent (TFA:PhSMe:EDT:TIS:H₂O=84:5:5:5:1, (V/V)) was added at an amount of 8 ml/g (cleaving agent/resin). The resulting mixture was stirred for 2.5 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 341 g white powdery solid of lixisenatide crude peptide. MS (Figure 1) MALDI-TOF: (M+H)⁺=4859.216. The yield of the crude peptide was 99.4% and the purity detected by HPLC was 69.1%.

44 g of the lixisenatide crude peptide obtained in 1.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 12.6 g of lixisenatide refined peptide. The purity of the refined peptide detected by HPLC was 99.3% and the yield was 28.8%.

### Example 2

In this example, only dipeptide A, dipeptide B, dipeptide D, and dipeptide F were introduced to prepare lixisenatide.

### 2.1 Preparation of Fmoc-Lys(Boc)-Rink Amide Resin

356 g of dry Rink Amide Resin (substitution degree of 0.42 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 210.9 g of Fmoc-Lys(Boc)-OH (450 mmol), 63.8 g of HOBt (473 mmol) and 170.6 g of HBTU (450 mmol) were dissolved in a mixed solvent of DMF and DCM. After the amino acid was dissolved, 87.6 g (675 mmol) of DIPEA was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 390 g of Fmoc-Lys (Boc)-Rink Amide Resin with a substitution degree detected of 0.385 mmol/g.

### 2.2 Preparation of lixisenatide peptide resin

421 g (150 mmol) of Fmoc-Lys(Boc)-Rink Amide Resin with a substitution degree of 0.385 mmol/g prepared in method 2.1 was weighted and added into a solid-phase reaction column. The resin was washed twice with DMF, followed by swelling in DMF for 30 minutes. After the deprotection of Fmoc by DBLK, the resin was washed 6 times with DMF. 211.3 g (450 mmol) Fmoc-Lys(Boc)-OH, 64.8 g (473 mmol) HOBt, and 63.9 g (473 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-OH, Fmoc-Pro-Ser(PSI ME, ME Pro)-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME,ME Pro)-OH, Fmoc-Gly-Thr(PSI ME,ME Pro)-OH, Fmoc-Glu(OtBu)-OH, Fmoc-Gly-OH, and Boc-His(Trt)-OH. After the reaction was completed, the resin was shrunk by methanol and dried under vacuum overnight to give 1611 g of lixisenatide peptide resin.

### 2.3 Cleaving of lixisenatide peptide resin and preparation of refined peptide

600 g of lixisenatide peptide resin obtained in 2.2 was placed in a cleaving reactor and a cleaving agent (TFA:PhSMe:EDT:H₂O=91:3:3:3, (V/V)) was added in an amount of 10 ml/g (cleaving agent/resin). The resulting mixture was stirred for 2.5 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 251 g white powdery solid of lixisenatide crude peptide. MS MALDI-TOF: (M+H)⁺=4859.017. The yield of the crude peptide was 101.2% and the purity detected by HPLC was 66.6%.

47 g of the lixisenatide crude peptide obtained in 2.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 12.4 g of lixisenatide refined peptide. The purity of the refined peptide detected by HPLC was 99.1% and the yield was 26.4%.

### Example 3

In this example, only dipeptide A, dipeptide B, dipeptide D, and dipeptide E were introduced to prepare lixisenatide.

### 3.1 Preparation of Fmoc-Lys(Boc)-Siber Resin

155 g of dry Siber Resin (substitution degree of 0.35 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 77.6 g of Fmoc-Lys(Boc)-OH (165 mmol), and 23.5 g of HOBt (173 mmol) were dissolved in a mixed solvent of DMF and DCM. After the amino acid was dissolved, 22.1 g (173 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 368 g of Fmoc-Lys (Boc)-Siber Resin with a substitution degree detected of 0.323 mmol/g.

### 3.2 Preparation of lixisenatide peptide resin

368 g (54 mmol) of Fmoc-Lys(Boc)-Siber Resin with a substitution degree of 0.323 mmol/g prepared in method 3.1 was weighted and added into a solid-phase reaction column. The resin was washed twice with DMF, followed by swelling in DMF for 30 minutes. After the deprotection of Fmoc by DBLK, the resin was washed 6 times with DMF. 77.9 g (165 mmol) Fmoc-Lys(Boc)-OH, 23.5 g (173 mmol) HOBt, and 22.4 g (173 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH, and Boc-His(Trt)-OH. After the reaction was completed, the resin was shrunk by methanol and dried under vacuum overnight to give 831 g of lixisenatide peptide resin.

### 3.3 Cleaving of lixisenatide peptide resin and preparation of refined peptide

800 g of lixisenatide peptide resin obtained in 3.2 was placed in a cleaving reactor and a cleaving agent (TFA:PhSMe:EDT:PhOMe=90:5:3:2, (V/V)) was added in an amount of 12 ml/g (cleaving agent/resin). The resulting mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 241 g white powdery solid of lixisenatide crude peptide. MS MALDI-TOF: (M+H)⁺=4859.512. The yield of the crude peptide was 98.9% and the purity detected by HPLC was 68.4%.

50 g of the lixisenatide crude peptide obtained in 3.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 13.7 g of lixisenatide refined peptide. The purity of the refined peptide detected by HPLC was 99.2% and the yield was 27.4%.

### Example 4

In this example, only dipeptide B, dipeptide D, and dipeptide E were introduced to prepare lixisenatide.

### 4.1 Preparation of Fmoc-Lys(Boc)-Rink Amide-MBHA Resin

135 g of dry Rink Amide-MBHA Resin (substitution degree of 0.24 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 46.8 g of Fmoc-Lys(Boc)-OH (100 mmol), and 14.2 g of HOBt (105 mmol) were dissolved in a mixed solvent of DMF and DCM. After the amino acid was dissolved, 13.5 g (105 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 143 g of Fmoc-Lys (Boc)-Siber Resin with a substitution degree detected of 0.223 mmol/g.

### 4.2 Preparation of lixisenatide peptide resin

143 g (32 mmol) of Fmoc-Lys(Boc)-Rink Amide-MBHA Resin with a substitution degree of 0.223 mmol/g prepared in method 4.1 was weighted and added into a solid-phase reaction column. The resin was washed twice with DMF, followed by swelling in DMF for 30 minutes. After the deprotection of Fmoc by DBLK, the resin was washed 6 times with DMF. 45.6 g (96 mmol) Fmoc-Lys(Boc)-OH, 13.8 g (101 mmol) HOBt, and 12.7 g (101 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Thr(tBu)-OH, Fmoc-Gly-OH, Fmoc-Glu(OtBu)-OH, Fmoc-Gly-OH, and Boc-His(Trt)-OH. After the reaction was completed, the resin was shrunk by methanol and dried under vacuum overnight to give 401 g of lixisenatide peptide resin.

### 4.3 Cleaving of lixisenatide peptide resin and preparation of refined peptide

400 g of lixisenatide peptide resin obtained in 4.2 was placed in a cleaving reactor and a cleaving agent (TFA:PhSMe:EDT: PhOH:H₂O=85:5:3:5:2, (V/V)) was added in an amount of 15 ml/g (cleaving agent/resin). The resulting mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 156 g white powdery solid of lixisenatide crude peptide. MS MALDI-TOF: (M+H)⁺=4859.347. The yield of the crude peptide was 102.6% and the purity detected by HPLC was 67.9%.

46 g of the lixisenatide crude peptide obtained in 4.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 11.6 g of lixisenatide refined peptide. The purity of the refined peptide detected by HPLC was 99.3% and the yield was 27.1%.

### Example 5

In this example, only dipeptide C, and dipeptide E were introduced to prepare lixisenatide.

### 5.1 Preparation of Fmoc-Lys(Boc)-Rink Amide-AM Resin

547 g of dry Rink Amide-MBHA Resin (substitution degree of 0.56 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 431.2 g of Fmoc-Lys(Boc)-OH (918 mmol), and 130.5 g of HOBt (964 mmol) were dissolved in a mixed solvent of DMF and DCM. After the amino acid was dissolved, 121.5 g (964 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 621 g of Fmoc-Lys(Boc)-Rink Amide-AM Resin with a substitution degree detected of 0.508 mmol/g.

### 5.2 Preparation of lixisenatide peptide resin

207 g (105 mmol) of Fmoc-Lys(Boc)-Rink Amide-AM Resin with a substitution degree of 0.508 mmol/g prepared in method 5.1 was weighted and added into a solid-phase reaction column. The resin was washed twice with DMF, followed by swelling in DMF for 30 minutes. After the deprotection of Fmoc by DBLK, the resin was washed 6 times with DMF. 147.6 g (315 mmol) Fmoc-Lys(Boc)-OH, 44.8 g (330 mmol) HOBt, and 41.6 g (330 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Thr(tBu)-Ser(PSI ME,ME Pro)-OH, Fmoc-Phe--OH, Fmoc-Thr(tBu)-OH, Fmoc-Gly-OH, Fmoc-Glu(OtBu)-OH, Fmoc-Gly-OH, and Boc-His(Trt)-OH. After the reaction was completed, the resin was shrunk by methanol and dried under vacuum overnight to give 1046 g of lixisenatide peptide resin.

### 5.3 Cleaving of lixisenatide peptide resin and preparation of refined peptide

1000 g of lixisenatide peptide resin obtained in 5.2 was placed in a cleaving reactor and a cleaving agent (TFA: EDT: H₂O=90:5:5, (V/V)) was added in an amount of 10 ml/g (cleaving agent/resin). The mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 487 g white powdery solid of lixisenatide crude peptide. MS MALDI-TOF: (M+H)⁺=4859.109. The yield of the crude peptide was 100.6% and the purity detected by HPLC was 67.1%.

43 g of the lixisenatide crude peptide obtained in 5.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 11.9 g of lixisenatide refined peptide. The purity of the refined peptide detected by HPLC was 99.3% and the yield was 28.1%.

### Example 6:

In this example, only dipeptide E was introduced to prepare lixisenatide.

### 6.1 Preparation of Fmoc-Lys(Boc)-Rink Amide-AM Resin

547 g of dry Rink Amide-AM Resin (substitution degree of 0.56 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 431.2 g of Fmoc-Lys(Boc)-OH (918 mmol), and 130.5 g of HOBt (964 mmol) were dissolved in a mixed solvent of DMF and DCM. After the amino acid was dissolved, 121.5 g (964 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 621 g of Fmoc-Lys(Boc)-Rink Amide-AM Resin with a substitution degree detected of 0.508 mmol/g.

### 6.2 Preparation of lixisenatide peptide resin

137 g (70 mmol) of Fmoc-Lys(Boc)-Rink Amide-AM Resin with a substitution degree of 0.508 mmol/g prepared in method 6.1 was weighted and added into a solid-phase reaction column. The resin was washed twice with DMF, followed by swelling in DMF for 30 minutes. After the deprotection of Fmoc by DBLK, the resin was washed 6 times with DMF. 98.5 g (210 mmol) Fmoc-Lys(Boc)-OH, 30.6 g (220 mmol) HOBt, and 27.9 g (220 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH, and Boc-His(Trt)-OH. After the reaction was completed, the resin was shrunk by methanol and dried under vacuum overnight to give 728 g of lixisenatide peptide resin.

### 6.3 Cleaving of lixisenatide peptide resin and preparation of refined peptide

728 g of lixisenatide peptide resin obtained in 6.2 was placed in a cleaving reactor and a cleaving agent (TFA: EDT: H₂O=90:5:5, (V/V)) was added in an amount of 10 ml/g (cleaving agent/resin). The mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 338.9 g white powdery solid of lixisenatide crude peptide. MS MALDI-TOF: (M+H)⁺=4859.205. The yield of the crude peptide was 99.6% and the purity detected by HPLC was 61.8%.

58 g of the lipisatetide crude peptide obtained in 6.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 14.9 g of lixisenatide refined peptide. The purity of the refined peptide detected by HPLC was 99.2% and the yield was 25.8%.

### Example 7

In this example, only dipeptide D was introduced to prepare lixisenatide.

### 7.1 Preparation of Fmoc-Lys(Boc)-Rink Amide-AM Resin

547 g of dry Rink Amide-AM Resin (substitution degree of 0.56 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 431.2 g of Fmoc-Lys(Boc)-OH (918 mmol), and 130.5 g of HOBt (964 mmol) were dissolved in a mixed solvent of DMF and DCM. After the amino acid was dissolved, 121.5 g (964 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 621 g of Fmoc-Lys(Boc)-Rink Amide-AM Resin with a substitution degree detected of 0.508 mmol/g.

### 7.2 Preparation of lixisenatide peptide resin

137 g (70 mmol) of Fmoc-Lys(Boc)-Rink Amide-AM Resin with a substitution degree of 0.508 mmol/g prepared in method 7.1 was weighted and added into a solid-phase reaction column. The resin was washed twice with DMF, followed by swelling in DMF for 30 minutes. After the deprotection of Fmoc by DBLK, the resin was washed 6 times with DMF. 98.5 g (210 mmol) Fmoc-Lys(Boc)-OH, 31.6 g (220 mmol) HOAt, and 27.9 g (220 mmol) DIC were dissolved in a mixed solution of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH, and Boc-His(Trt)-OH. After the reaction was completed, the resin was shrunk by methanol and dried under vacuum overnight to give 742 g of lixisenatide peptide resin.

### 7.3 Cleaving of lixisenatide peptide resin and preparation of refined peptide

742 g of lixisenatide peptide resin obtained in 7.2 was placed in a cleaving reactor and a cleaving agent (TFA: PhSMe: PhOH: EDT: H₂O=85:5:3:5:2, (V/V)) was added in an amount of 10 ml/g (cleaving agent/resin). The resulting mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 341.3 g white powdery solid of lixisenatide crude peptide. MS MALDI-TOF: (M+H)⁺=4859.318. The yield of the crude peptide was 100.8% and the purity detected by HPLC was 59.6%.

48 g of the lipisatetide crude peptide obtained in 7.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 12.8 g of lixisenatide refined peptide. The purity of the refined peptide detected by HPLC was 99.1% and the yield was 26.6%.

### Example 8

In this example, only dipeptide A, dipeptide B, dipeptide D, dipeptide E, and dipeptide F were introduced to prepare lixisenatide.

### 8.1 Preparation of Fmoc-Lys(Boc)-Rink Amide-AM Resin

547 g of dry Rink Amide-AM Resin (substitution degree of 0.56 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 431.2 g of Fmoc-Lys(Boc)-OH (918 mmol), and 130.5 g of HOBt (964 mmol) were dissolved in a mixed solvent of DMF and DCM. After the amino acid was dissolved, 121.5 g (964 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 621 g of Fmoc-Lys(Boc)-Rink Amide-AM Resin with a substitution degree detected of 0.508 mmol/g.

### 8.2 Preparation of lixisenatide peptide resin

136 g (70 mmol) of Fmoc-Lys(Boc)-Rink Amide-AM Resin with a substitution degree of 0.508 mmol/g prepared in method 8.1 was weighted and added into a solid-phase reaction column. The resin was washed twice with DMF, followed by swelling in DMF for 30 minutes. After the deprotection of Fmoc by DBLK, the resin was washed 6 times with DMF. 98.5 g (210 mmol) Fmoc-Lys(Boc)-OH, 31.6 g (220 mmol) HOAt, 109.5 g (210 mmol) of PyAOP, and 54.3 g (420 mmol)) of DIPEA were dissolved in a mixture of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME,ME Pro)-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME,ME Pro)-OH, Fmoc-Gly-Thr(PSI ME,ME Pro)-OH, Fmoc-Glu(OtBu)-OH, Fmoc-Gly-OH, and Boc-His(Trt)-OH. After the reaction was completed, the resin was shrunk by methanol and dried under vacuum overnight to give 751 g of lixisenatide peptide resin.

### 8.3 Cleaving of lixisenatide peptide resin and preparation of refined peptide

742 g of lixisenatide peptide resin obtained in 8.2 was placed in a cleaving reactor and a cleaving reagent (TFA: PhSMe: PhOH: EDT: H₂O=85:5:3:5:2, (V/V)) was added in an amount of 10 ml/g (cleaving reagent/resin). The resulting mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 335.6 g white powdery solid of lixisenatide crude peptide. MS MALDI-TOF: (M+H)⁺=4859.478. The yield of the crude peptide was 97.6% and the purity detected by HPLC was 68.9%.

52 g of the lipisatetide crude peptide obtained in 8.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 14.5 g of lixisenatide refined peptide. The purity of the refined peptide detected by HPLC was 99.2% and the yield was 27.9%.

### Comparative Example 1

### D1.1 Preparation of Fmoc-Lys(Boc)-Rink Amide-AM Resin

410 g of dry Rink Amide-AM Resin (substitution degree of 0.56 mmol/g) was weighted and added into a solid-phase reaction column. The resin was first washed twice with DMF, followed by swelling in 2-3 times the resin bed volume of DMF for 30 minutes. The resin washed three times with DMF and then twice with DCM and left for feeding.

Under the condition of ice bath, 323.5 g of Fmoc-Lys(Boc)-OH (690 mmol), and 97.9 g of HOBt (725 mmol) were dissolved in a mixed solvent of DMF and DCM. After the amino acid was dissolved, 92.4 g (725 mmol) of DIC was slowly added and activated for 3 minutes. The resulting reaction solution was poured into the reaction column, and the reaction was carried out under stirring with air-blowing. The reaction was stopped when ninhydrin test shown that the resin was transparent. The resin was washed 3 times with DMF and shrunk by methanol. The resin was dried under reduced pressure to give 462 g of Fmoc-Lys(Boc)-Rink Amide-AM Resin with a substitution degree detected of 0.51 mmol/g.

### D1.2 Preparation of lixisenatide peptide resin via sequential coupling

462 g (230 mmol) of Fmoc-Lys(Boc)-Rink Amide-AM Resin with a substitution degree of 0.51 mmol/g prepared in method D1.1 was weighted and added into a solid-phase reaction column. The resin was washed twice with DMF, followed by swelling in DMF for 30 minutes. After the deprotection of Fmoc by DBLK, the resin was washed 6 times with DMF. 323.6 g (690 mmol) Fmoc-Lys(Boc)-OH, 98.1 g (725 mmol) HOBt, and 92.4 g (724 mmol) DIC were dissolved in a mixture of DCM and DMF (volume ratio of 1:1), and then added into the solid-phase reaction column and allowed to react at room temperature for 2h (the completion of the reaction was determined by ninhydrin test; if the resin was colorless and transparent, the reaction was complete; if the resin was colored, the reaction was incomplete, and in this case, the coupling reaction was carried out for additional 1h until the resin detected was transparent).

The steps of deprotecting Fmoc and coupling of corresponding amino acid added were repeated, and the following coupling was completed according to the order of the sequence by using certain coupling method: Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH, and Boc-His(Trt)-OH. After the reaction was completed, the resin was shrunk by methanol and dried under vacuum overnight to give 2350 g of lixisenatide peptide resin.

### D1.3 Cleaving of lixisenatide peptide resin and preparation of refined peptide

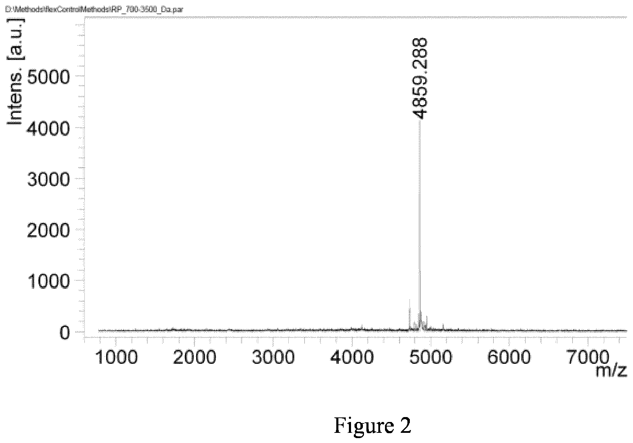

800 g of lixisenatide peptide resin obtained in D1.2 was placed in a cleaving reactor and a cleaving agent (TFA: PhSMe: PhOH: EDT: H₂O=85:5:3:5:2, (V/V)) was added in an amount of 12 ml/g (cleaving agent/resin). The resulting mixture was stirred for 3 h at room temperature and then filtered through a sand core funnel, and the filtrate was collected. The resin was washed twice with a small amount of TFA. The filtrates were combined and concentrated under reduced pressure. Ice-cold anhydrous ether was added to the filtrates for precipitation followed by centrifugation. The cake of the crude peptide was washed three times with anhydrous ether and dried under vacuum to give 370.6 g white powdery solid of lixisenatide crude peptide. MS (Fig. 2) MALDI-TOF: (M+H)⁺=4859.288. The yield of the crude peptide was 96.8% and the purity detected by HPLC was 44.5%.

49 g of the lipisatetide crude peptide obtained in D1.3 was dissolved and subjected to refining by using NOVASEP RP-HPLC system under conditions: wavelength 220 nm, reversed-phase C18 column. The lixisenatide was purified by using a normal 0.1% TFA/water and acetonitrile mobile phase system and salt conversion was performed. The fractions of the peak of interest were collected, concentrated by rotary evaporation and lyophilized to give 7.5 g of lixisenatide refined peptide. The purity of the refined peptide detected by HPLC was 98.6% and the yield was 15.4%.

### Example 9

The refined peptides prepared in examples 1 to 8 and Comparative Example 1 were tested. The mass spectrum of the crude peptide obtained in Example 1 is shown in Figure 1, and the mass spectrum of the crude peptide obtained in Comparative Example 1 is shown in Figure 2. The mass spectra of the products obtained in the other examples are similar. The purity and yield of the polypeptide obtained in each example are shown in Table 2:

**Table 2. Purity and yield of polypeptide**

| | MS MALDI-TOF: (M+H)⁺ | Yield of Crude Peptide | Purity of Crude Peptide | Yield of Refined Peptide | Purity of Refined Peptide |
|---|---|---|---|---|---|
| Example 1 | 4859.216 | 99.4% | 69.1% | 28.8% | 99.3% |
| Example 2 | 4859.017 | 101.2% | 66.6% | 26.4% | 99.1% |
| Example 3 | 4859.512 | 98.9% | 68.4% | 27.4% | 99.2% |
| Example 4 | 4859.347 | 102.6% | 67.9% | 27.1% | 99.3% |
| Example 5 | 4859.109 | 100.6% | 67.1% | 28.1% | 99.3% |
| Example 6 | 4859.205 | 99.6% | 61.8% | 25.8% | 99.2% |
| Example 7 | 4859.318 | 100.8% | 59.6% | 26.6% | 99.1% |
| Example 8 | 4859.478 | 97.6% | 68.9% | 27.9% | 99.2% |
| Comparative Example 1 | 4859.288 | 96.8% | 44.5% | 15.4% | 98.6% |

The results show that the technical solution of introducing dipeptide segment to the synthesis method is better than the method of comparative example in terms of the purity of the crude peptide, the purity of the refined peptide, and the yield of the refined peptide.

## Claims

1. A method for preparing lixisenatide, comprising:
step 1: synthesizing Fmoc-Lys-resin by solid-phase synthesis ;
step 2: coupling an amino acid and a dipeptide to the Fmoc-Lys-resin according to the peptide sequence of lixisenatide to obtain lixisenatide peptide resin; wherein the dipeptide is selected from the group consisting of -Gly-Thr, -Phe-Thr, -Thr-Ser, -Leu-Ser, -Ser-Ser and -Pro-Ser; and
step 3: cleaving the lixisenatide peptide resin to obtain lixisenatide,
wherein
coupling -Gly-Thr is performed by using Fmoc-Gly-Thr(PSI ME, ME Pro)-OH;
coupling -Phe-Thr is performed by using Fmoc-Phe-Thr(PSI ME, ME Pro)-OH;
coupling -Thr-Ser is performed by using Fmoc-Thr(tBu)-Ser(PSI ME, ME Pro)-OH;
coupling -Leu-Ser is performed by using Fmoc-Leu-Ser(PSI ME, ME Pro)-OH;
coupling -Ser-Ser is performed by using Fmoc-Ser(tBu)-Ser(PSI ME, ME Pro)-OH; and
coupling -Pro-Ser is performed by using Fmoc-Pro-Ser(PSI ME, ME Pro)-OH.

2. The method according to claim 1, wherein the coupling agent for the coupling is a mixture of HOBt and DIC; and wherein the molar ratio of HOBt to DIC is 1:1.

3. The method according to claim 1, wherein the cleaving agent for the cleaving comprises TFA and component B; and wherein the component B is selected from the group consisting of PhSMe, PhOMe, EDT, H₂O, TIS and PhOH.

4. The method according to claim 1, after the step 3, further comprising steps of purification and salt conversion.

5. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME , ME Pro)-OH, Fmoc-Pro-OH, Fmoc-Gly-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME , ME Pro)-OH, Fmoc-Asp(OtBu)-OH, Fmoc-Thr(tBu)-Ser(PSI ME, ME Pro)-OH, Fmoc-Phe-OH, Fmoc-Gly-Thr(PSI ME, ME Pro)-OH, Fmoc-Glu(OtBu)-OH, Fmoc-Gly-OH and Boc-His(Trt)-OH.

6. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME,ME Pro)-OH, Fmoc-Pro-OH, Fmoc-Ala-OH, Fmoc-Gly-OH, Fmoc-Ser(tBu)-OH, Fmoc-Pro-Ser(PSI ME,ME Pro)-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH and Boc-His(Trt)-OH.

7. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH and Boc-His(Trt)-OH.

8. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME,ME Pro)-OH, Fmoc-Asp(OtBu)-OH, Fmoc-Ser(tBu)-OH, Fmoc-Phe-Thr(PSI ME, ME Pro)-OH, Fmoc-Thr(tBu)-OH, Fmoc-Gly-OH, Fmoc-Glu(OtBu)-OH, Fmoc-Gly-OH and Boc-His(Trt)-OH.

9. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Thr(tBu)-Ser(PSI ME, ME Pro)-OH, Fmoc-Phe--OH, Fmoc-Thr(tBu)-OH, Fmoc-Gly-OH, Fmoc-Glu(OtBu)-OH, Fmoc-Gly-OH and Boc-His(Trt)-OH.

10. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH and Boc-His(Trt)-OH.

11. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Leu-OH, Fmoc-Asp(OtBu)-OH, Fmoc-Ser(tBu)-OH, Fmoc-Thr(tBu)-OH, Fmoc-Phe--OH, Fmoc-Thr(tBu)-OH, Fmoc-Gly-OH, Fmoc-Glu(OtBu)-OH, Fmoc-Gly-OH or Boc-His(Trt)-OH.

12. The method according to claim 1, wherein the coupling is performed by coupling sequentially the following: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME,ME Pro)-OH, Fmoc-Pro-OH, Fmoc-Ala-OH, Fmoc-Gly-OH, Fmoc-Ser(tBu)-Ser(PSI ME,ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH and Boc-His(Trt)-OH.

## Patentansprüche

1. Verfahren zur Herstellung von Lixisenatid, umfassend:
Schritt 1: Synthetisieren von Fmoc-Lys-Harz durch Festphasensynthese;
Schritt 2: Koppeln einer Aminosäure und eines Dipeptids mit dem Fmoc-Lys-Harz gemäß der Peptidsequenz von Lixisenatid, um Lixisenatid-Peptidharz zu erhalten; wobei das Dipeptid ausgewählt ist aus der Gruppe bestehend aus -Gly-Thr, -Phe-Thr, - Thr-Ser, -Leu-Ser, -Ser-Ser und -Pro-Ser; und
Schritt 3: Spalten des Lixisenatid-Peptidharzes, um Lixisenatid zu erhalten;
wobei
das Koppeln von -Gly-Thr unter Verwendung von Fmoc-Gly-Thr(PSI ME, ME Pro)-OH durchgeführt wird;
das Koppeln von -Phe-Thr unter Verwendung von Fmoc-Phe-Thr(PSI ME, ME Pro)-OH durchgeführt wird;
das Koppeln von -Thr-Ser unter Verwendung von Fmoc-Thr(tBu)-Ser(PSI ME, ME Pro)-OH durchgeführt wird;
das Koppeln von -Leu-Ser unter Verwendung von Fmoc-Leu-Ser(PSI ME, ME Pro)-OH durchgeführt wird;
das Koppeln von -Ser-Ser unter Verwendung von Fmoc-Ser(tBu)-Ser(PSI ME, ME Pro)-OH durchgeführt wird; und
das Koppeln von -Pro-Ser unter Verwendung von Fmoc-Pro-Ser(PSI ME, ME Pro)-OH durchgeführt wird.

2. Verfahren nach Anspruch 1, wobei das Kopplungsmittel für die Kopplung ein Gemisch aus HOBt und DIC ist; und wobei das Molverhältnis von HOBt zu DIC 1:1 beträgt.

3. Verfahren nach Anspruch 1, wobei das Spaltmittel für die Spaltung TFA und Komponente B umfasst; und wobei die Komponente B ausgewählt ist aus der Gruppe bestehend aus PhSMe, PhOMe, EDT, H₂O, TIS und PhOH.

4. Verfahren nach Anspruch 1, das nach Schritt 3 ferner die Schritte der Aufreinigung und der Salzumwandlung umfasst.

5. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Thr(tBu)-Ser(PSI ME, ME Pro)-OH, Fmoc-Phe-OH, Fmoc-Gly-Thr(PSI ME, ME Pro)-OH, Fmoc-Glu(OtBu)-OH, Fmoc-Gly-OH und Boc-His(Trt)-OH.

6. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-OH, Fmoc-Pro-Ser(PSI ME, ME Pro)-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH und Boc-His(Trt)-OH.

7. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH und Boc-His(Trt)-OH.

8. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Thr(tBu)-OH, Fmoc-Gly-OH, Fmoc-Glu(OtBu)-OH, Fmoc-Gly-OH und Boc-His(Trt)-OH.

9. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Thr(tBu)-Ser(PSI ME, ME Pro)-OH, Fmoc-Phe-OH, Fmoc-Thr(tBu)-OH, Fmoc-Gly-OH, Fmoc-Glu(OtBu)-OH, Fmoc-Gly-OH und Boc-His(Trt)-OH.

10. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH und Boc-His(Trt)-OH.

11. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH und Boc-His(Trt)-OH.

12. Verfahren nach Anspruch 1, wobei die Kopplung durch sequentielles Koppeln von folgendem durchgeführt wird: Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH und Boc-His(Trt)-OH.

## Revendications

1. Procédé pour préparer du lixisénatide, comprenant :
étape 1 : la synthèse de Fmoc-Lys-résine par synthèse en phase solide ;
étape 2 : le couplage d'un acide aminé et d'un dipeptide à la Fmoc-Lys-résine selon la séquence peptidique du lixisénatide afin d'obtenir le peptide lixisénatide résine, le dipeptide étant choisi dans le groupe constitué par -Gly-Thr, -Phe-Thr, -Thr-Ser, -Leu-Ser, -Ser-Ser et -Pro-Ser ; et
étape 3 : le clivage du peptide lixisénatide résine pour obtenir du lixisénatide,
dans lequel
le couplage -Gly-Thr est réalisé en utilisant Fmoc-Gly-Thr(PSI ME, ME Pro)-OH ;
le couplage -Phe-Thr est réalisé en utilisant Fmoc-Phe-Thr(PSI ME, ME Pro)-OH;
le couplage -Thr-Ser est réalisé en utilisant Fmoc-Thr(tBu)-Ser(PSI ME, ME Pro)-OH;
le couplage -Leu-Ser est réalisé en utilisant Fmoc-Leu-Ser(PSI ME, ME Pro)-OH;
le couplage -Ser-Ser est réalisé en utilisant Fmoc-Ser(tBu)-Ser(PSI ME, ME Pro)-OH ; et
le couplage -Pro-Ser est réalisé en utilisant Fmoc-Pro-Ser(PSI ME, ME Pro)-OH.

2. Procédé selon la revendication 1, dans lequel l'agent de couplage pour le couplage est un mélange d'HOBt et de DIC ; et dans lequel le rapport molaire entre HOBt et DIC est de 1:1.

3. Procédé selon la revendication 1, dans lequel l'agent de clivage pour le clivage comprend du TFA et un composant B ; et dans lequel le composant B est choisi dans le groupe constitué par PhSMe, PhOMe, EDT, H₂O, TIS et PhOH.

4. Procédé selon la revendication 1, après l'étape 3, comprenant en outre des étapes de purification et de conversion de sel.

5. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Thr(tBu)-Ser(PSI ME, ME Pro)-OH, Fmoc-Phe-OH, Fmoc-Gly-Thr(PSI ME, ME Pro)-OH, Fmoc-Glu(OtBu)-OH, Fmoc-Gly-OH et Boc-His(Trt)-OH.

6. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-OH, Fmoc-Pro-Ser(PSI ME, ME Pro)-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH et Boc-His(Trt)-OH.

7. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH et Boc-His(Trt)-OH.

8. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Thr(tBu)-OH, Fmoc-Gly-OH, Fmoc-Glu(OtBu)-OH, Fmoc-Gly-OH et Boc-His(Trt)-OH.

9. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Thr(tBu)-Ser(PSI ME, ME Pro)-OH, Fmoc-Phe-OH, Fmoc-Thr(tBu)-OH, Fmoc-Gly-OH, Fmoc-Glu(OtBu)-OH, Fmoc-Gly-OH et Boc-His(Trt)-OH.

10. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Ser(tBu)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH et Boc-His(Trt)-OH.

11. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Ser(tBu)-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Leu-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, Fmoc-Gly-OH ou Boc-His(Trt)-OH.

12. Procédé selon la revendication 1, dans lequel le couplage est réalisé par couplage séquentiel des éléments suivants : Fmoc-Lys(Boc)-OH, Fmoc-Lys(Boc)-OH, Fmoc-Lys(Boc)-OH, Fmoc-Lys(Boc)-OH, Fmoc-Lys(Boc)-OH, Fmoc-Pro-Ser(PSI ME, ME Pro)-OH, Fmoc-Pro-OH, Fmoc-Ala-OH, Fmoc-Gly-OH, Fmoc-Ser(tBu)-Ser(PSI ME, ME Pro)-OH, Fmoc-Pro-OH, Fmoc-Gly-OH, Fmoc-Gly-OH, Fmoc-Asn(Trt)-OH, Fmoc-Lys(Boc)-OH, Fmoc-Leu-OH, Fmoc-Trp(Boc)-OH, Fmoc-Glu(OtBu)-OH, Fmoc-Ile-OH, Fmoc-Phe-OH, Fmoc-Leu-OH, Fmoc-Arg(Pbf)-OH, Fmoc-Val-OH, Fmoc-Ala-OH, Fmoc-Glu(OtBu)-OH, Fmoc-Glu(OtBu)-OH, Fmoc-Glu(OtBu)-OH, Fmoc-Met-OH, Fmoc-Gln(Trt)-OH, Fmoc-Lys(Boc)-OH, Fmoc-Leu-Ser(PSI ME, ME Pro)-OH, Fmoc-Asp(OtBu)-OH, Fmoc-Ser(tBu)-OH, Fmoc-Phe-Thr(PSI ME, ME Pro)-OH, Fmoc-Gly-Thr(PSI ME, ME Pro)-OH, Fmoc-Glu(OtBu)-OH, Fmoc-Gly-OH et Boc-His(Trt)-OH.
